# EUROPEAN PATENT SPECIFICATION

(11) **EP 2 626 017 B1**
(45) Date of publication and mention of the grant of the patent: **04.10.2017**
(21) Application number: 13155000.6
(22) Date of filing: 13.02.2013
(51) Int. Cl.: A61B 17/16

(54) **A reamer handle for reaming the pelvis (acetabulum) in minimal invasive orthopedic surgery**
Ahlenhandgriff zur Aufweitung des Beckens (Acetabulum) bei der minimalinvasiven orthopädischen Chirurgie
Poignée à alésoir pour aléser le bassin (acétabule) en chirurgie orthopédique avec effraction minimale

(30) Priority: 13.02.2012 TR 201201556
(43) Date of publication of application: 14.08.2013
(73) Proprietor: Sayan Tibbi Aletler Pazarlama Sanayi ve Ticaret Limited Sirketi, Izmir (TR)
(72) Inventor: Goren, Serdar Ömür, Izmir (TR)

(56) References cited:
- EP-A1- 1 442 729
- US-A1- 2005 038 443
- US-A1- 2007 293 869

## Description

### Technical Field

The invention is related to an apparatus to be used in minimal invasive orthopedic surgery.

The invention is particularly related to a reamer handle to be used for moving the hemispherical reamers of different diameters, changing in accordance with the bone size, for reaming the pelvis (acetabulum) in minimal invasive orthopedic surgery.

### State of the Art

In our day the most important disadvantages of the reamer handles used in minimal invasive surgeries are as follows:
The holding angle of the surgical motor connected to the apparatus for moving the reamer by the reamer handle of the apparatus is not ergonomic. The connection of the surgical motor of the apparatus is possible only on the center axis of the apparatus.

As it should be washed by being dismantled completely to be cleaned after surgery the apparatus may completely or partially become unusable because some tiny parts of the inner mechanism may be lost during the washing or mounting processes.

Prior art provides a handle for the physician to hold the apparatus. For the holding positions apart from this the physicians need to develop their own alternatives.

The apparatus doesn't provide practical possibilities for using different surgical motors.

As it is completely detachable the manufacturing method thereof is much more complicated and therefore the manufacturing costs are high.

In the literature, one of the prior arts on the subject matter is the patent application numbered EP1442729 A1. The application is about a positioning tool for a joint socket cutting instrument or an implant, designed for use with a minimally invasive surgical procedure in conjunction with a computer assisted surgical procedure. The positioning tool has a longitudinally extending drive shaft having a moveable joint at a first end and a drive coupling for connecting to a power source at a second end. A holder for mounting a cutting tool such as a drill or an acetabular cutting instrument or for mounting an acetabular implant is coupled to the moveable joint at the first end of the drive shaft for movement with respect to the drive shaft. However, in the related application, the sufficient variety of angles in transferring movement from the surgical motor cannot be provided and the handle cannot be fixed at a desired angle in order to provide ease of use for the user. Additionally, the parts of the cited application may result in problems during the cleaning process since they are not detachable.

As a result, in order to move the hemispherical reamers of different diameters changing in accordance with the bone size, for reaming the pelvis (acetabulum) in minimal invasive orthopedic surgery, a development in the related art has to be made due to the requirement of a reamer handle which will overcome the inadequacy of the present solutions.

### Object of the Invention

In order to overcome the disadvantages of the state of the art one object of the present invention is to provide an easy adjustment of the handle for the physicians who use either his/her left hand or his/her right hand by connecting the handle to the reamer handle body via a lockable movable hinge and by transferring the movement of the surgical motor movement transfer shaft to the body shaft over the hinge.

Another object of the invention is to provide a stabilization and usage opportunity of the surgical motor connecting shaft of the apparatus relative to the body axis in a desired different angle value for the physicians. Thanks to the connection of the motor shaft and the handle to each other a complete control of the apparatus is provided by holding the apparatus securely in the chosen position for the usage of the motor shaft.

Another object of the invention is to increase the angular capability of the motor connecting shaft up to 63,5° (63° 30') relative to the body axis of the apparatus via a lockable adjustment lever with radius positioned inside the handle.

Another object of the invention is to demount the handle from the body easily and to provide the usage of the motor connecting shaft with a tool normal clearance angle between 29° and 81° on the vertical plane and between 29° and 67° on the horizontal plane relative to the body axis of the apparatus by removing the handle if necessary via a holder placed onto the body of the apparatus. During the usage of the tool normal clearance angle the control of the apparatus is provided by the holder on the body.

Another object of the invention is that it is constructed in such a way that the movement-transfer components including the surgical motor connection and the reamer for reaming the bone do not have to be demounted in order to clean the apparatus easily after the surgery and to eliminate the risk of the disuse of the components due to being lost during cleaning and during the mounting processes after the cleaning. The stable movement transfer member on the end of the apparatus used inside of the human body is protected in order not to give rise to an additional injury as a result of surrounding tissue. There are formed reciprocal channels thereon for a proper cleaning after the surgery. Same kinds of channels are opened on the handle for a proper cleaning of the adjustment lever mechanism with radius.

Another object of the invention is to use the apparatus easily with any kind of surgical motors via the adaptors connected to the motor connecting shaft easily.

The structural and the characteristic features and all of the advantages of the invention will be understood clearly from the figures and from the detailed description made in reference to the figures; and therefore the examination should be done by taking these figures and the detailed description into account.

### Figures Helping the Invention to be Understood

Fig 1 is the side view of the embodiment of the invention without the handle,
Fig 2 is a wide-angle top view of the embodiment of the invention without the handle,
Fig 3 is a narrow-angle top view of the embodiment of the invention without the handle,
Fig 4 is the side view of the embodiment of the invention with the handle,
Fig 5 is a wide-angle top view of the embodiment of the invention with the handle,
Fig 6 is a narrow-angle top view of the embodiment of the invention with the handle,
Fig 7 is a perspective view of the disassembled surgical motor adaptor and the embodiment of the invention with the handle.

It is not a necessity to scale the drawings and the details not necessary for understanding of the present invention have been omitted. Additionally, the elements which are at least substantially identical and which have at least substantially identical functions are indicated with the same number.

**Description of the Part References**

| | | | |
|---|---|---|---|
| **1.** | Body | **7.** | Adjusting lever with radius |
| **2.** | Handle fixing hinge | **8.** | Handle |
| **3.** | Motor connecting shaft | **9.** | Holder |
| **4.** | Stable movement transfer member | **10.** | Channels |
| **5.** | Movable hinge | **11.** | Reamer connection head |
| **6.** | Surgical motor adaptor | **12.** | Adjusting lever lock with radius |

### Detailed Description of the Invention

The invention is a reamer handle for transferring the movement from a surgical motor to a reamer used for reaming the pelvis (acetabulum) via a reamer connection head (11) in order to be used for driving reamers of different diameters, changing in accordance with the bone size, for reaming the pelvis (acetabulum) in minimal invasive orthopedic surgery.

The invention is defined in claim 1, with further embodiments defined in the dependent claims.

It is characterized in that it comprises:
an adjustment lever lock with radius (12) enabling said adjustment lever with radius (7) to be fixed in a desired position.

### The Preferred Embodiments of the Invention:

In this detailed description the preferred embodiments of the reamer handle of the present invention are disclosed only for the better understanding thereof and should not be considered as limiting the scope of the invention.

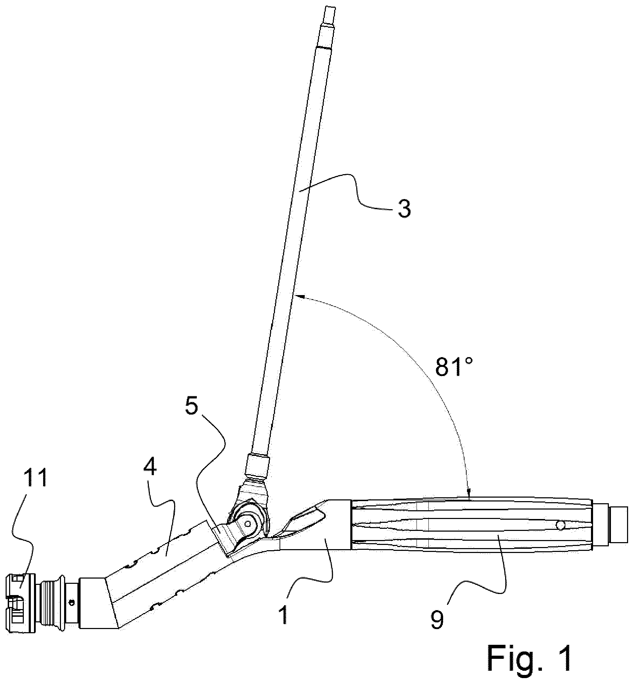

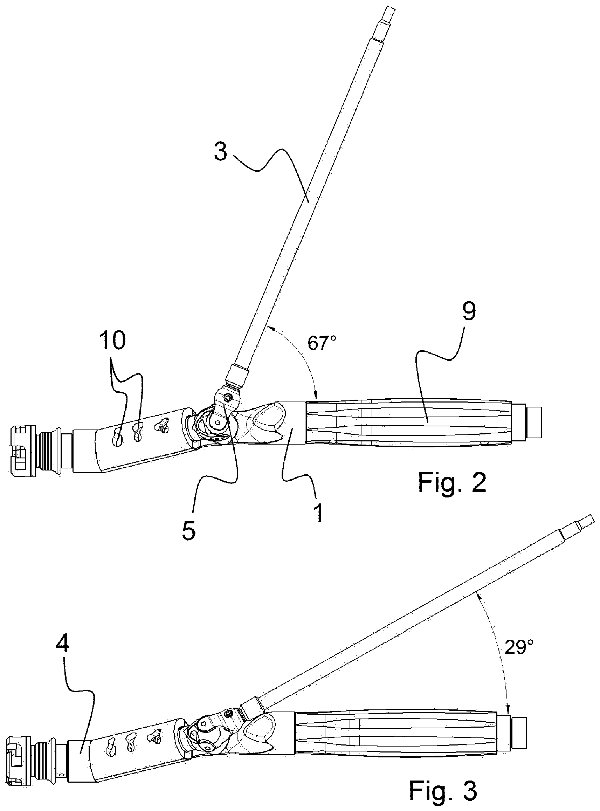

In Fig. 1 a side view of the preferred embodiment of the present invention is shown. The reamer handle according to the present invention generally serves as a transfer member which transfers the movement from the surgical motor to the reamer used for reaming the pelvis (acetabulum). During this function the movement is transferred to the stable shafts on the body (1) by a hinge (5). Therefore, the reamer handle has the capability of transferring movements in a desired angle within the predetermined limits through a movable hinge (5) (Figures 1-6). The invention comprises a holder (9) connected to the body (1). Thanks to the holder (9) providing a grip convenience particularly due to the recesses and the projections the invention can be hold securely and can be used easily.

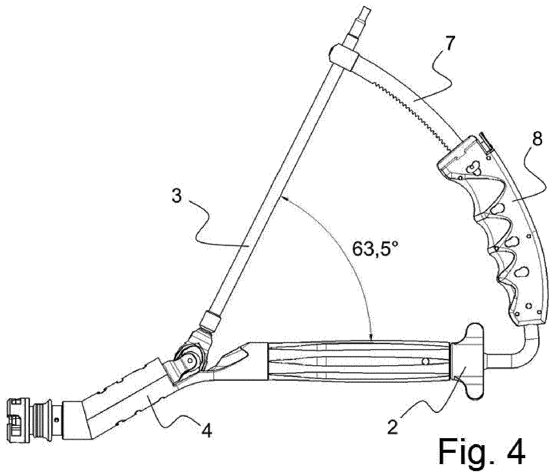

In addition to the above mentioned features the invention comprises a handle (8) which is connected to said body (1), which provides the holding of the reamer handle along a different axis from the body axis and which is connected onto said motor connecting shaft (3) (Figures 4-6). There is also a handle fixing hinge (2) which enables said handle (8) to be at different angles relative to the body and which stabilizes it on the body (1) at the desired angle. Said handle stabilization hinge (2) is located on said holder (9).

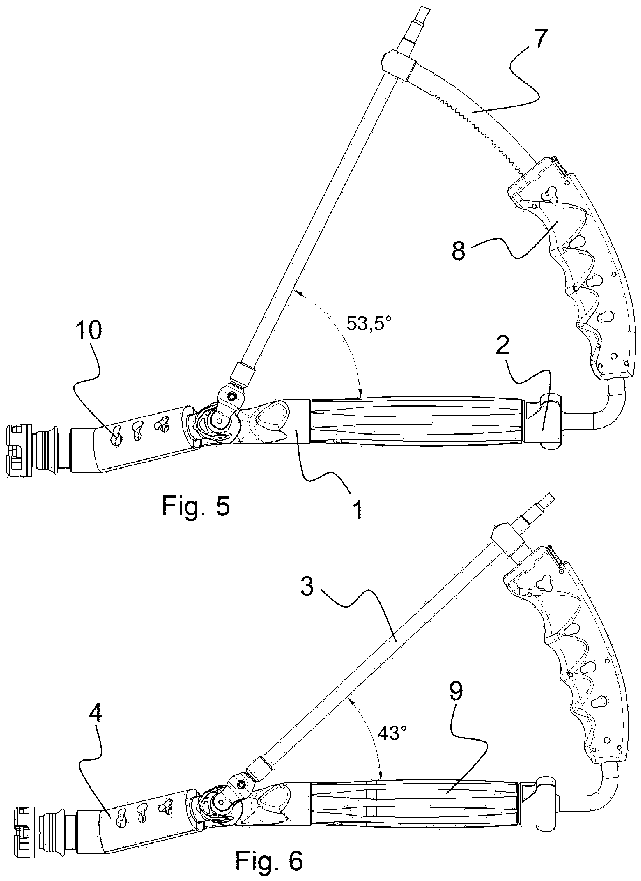

The double movable hinges (5) seen in the figures enables the physicians to use the surgical motor connection shaft (3) with a tool normal clearance angle of 43° relative to the body axis of the apparatus (Figure 6). Thanks to the mounting of the motor connection shaft (3) to the handle (8) the control of the apparatus is provided by holding the apparatus securely in the desired position to use the motor connection shaft (3). The movable hinge (5) transfers the movement to the stable movement transfer member (4) at different angles of the motor connection shaft (3).

There is also a lockable adjustment lever with radius (7) which is placed inside the handle (8) seen in Figures 4 and 5, which is removable and whose end outside the handle (8) is connected to said motor connection shaft (3). The angle adjustment capability of the motor connection shaft (3) is increased up to 63,5° relative to the body axis of the apparatus via this lever (7) (Figure 4). Therefore, when the adjustment lever with radius (7) is in the open position a 63,5° tool normal clearance angle on the vertical plane and a 53,5° of tool normal clearance on the horizontal plane relative to the apparatus, stabilization and usage of the motor connection shaft (3) is provided. In Figures 4-6 an adjustment lever lock with radius (12) fixing the adjustment lever with radius (7) in the desired position is shown. This lock (12) is preferably placed onto the handle (8) projecting from the adjustment lever (7).

As shown in the figures the stable movement transfer member (4) on the end of the apparatus used inside of the human body is protected in order not to give rise to an additional injury as a result of surrounding tissue. There are formed reciprocal channels (10) on said stable movement transfer member (4) for a proper cleaning after the surgery. Similarly, channels (10) are opened on the handle (8) for a proper cleaning of the adjustment lever mechanism with radius (7).

As shown in Figure 1 the surgical motor connection shaft (3) may be provided at a greater angle (81 degrees) in free usage on the vertical plane as the handle (8) has a detachable structure. By this means it provides an easier movement for the user physician during the surgery compared to the existing reamer handles. If desired, the motor connection shaft (3) may be used with a tool normal clearance angle between 29° and 67° on the horizontal plane and between 29° and 81° on the vertical plane relative to the body axis of the apparatus by removing the handle (8) (Figures 1-3). The control of the apparatus is provided via a holder (9) on the body (1) during the usage of the tool normal clearance angle.

With the reamer handle of the present invention the risk of malfunction of the apparatus is minimized which resulted from lost components encountered often as it cannot be separated into tiny parts even when the system is detached and as the components will not be missing during the cleaning after the surgery.

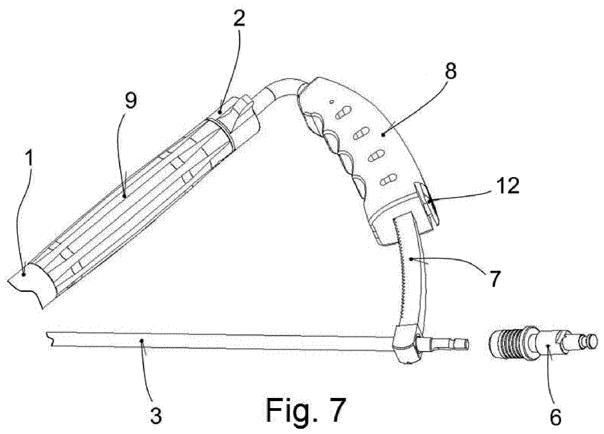

The present invention may be used easily with different surgical motors by the adaptors (6) to be mounted easily to the motor connection shaft (3) (Figure 7). As the movement of the surgical motor connection shaft (3) is transferred to the body (1) via a hinge (5) and the apparatus can be cleaned without being detached it may be used in the design of the apparatus to place the metal implant to be implanted after the reaming process of the acetabulum by a reamer.

## Claims

1. A reamer handle for transferring the movement from a surgical motor to a reamer used for reaming the pelvis (acetabulum) via a reamer connection head (11), in order to be used for driving reamers of different diameters, changing in accordance with the bone size, for reaming the pelvis (acetabulum) in minimal invasive orthopedic surgery; comprising a body (1); a reamer connection head (11) to which a reamer for use inside a human body can be connected; a stable movement transfer member (4) which connects the body (1) and the reamer connection head (11) and which is configured to transfer the movement from the motor to said reamer connection head (11); a motor connection shaft (3) for transferring the movement from the motor to said stable movement transfer member (4); a movable hinge (5) enabling the motor connection shaft (3) to be used at a tool normal clearance angle in the vertical plane and the horizontal plane relative to the body axis of the apparatus for transferring the movement of said motor connection shaft (3) at different angle values to the stable movement transfer member (4); a handle (8) which is connected to said body (1), and which enables the reamer handle to be held along a different axis than the body axis and which can be connected onto said motor connection shaft (3); an adjustment lever with radius (7) which is placed into said handle (8) in order to increase the movement easiness of said motor connection shaft (3), and which is removable and whose end outside the handle (8) can be connected to said motor connection shaft (3); **characterized in that** it comprises an adjustment lever lock with radius (12) enabling said adjustment lever with radius (7) to be fixed in a desired position.

2. A reamer handle according to claim 1, **characterized in that** it comprises a holder (9) connected to said body (1).

3. A reamer handle according to claim 1, **characterized in that** it comprises a handle fixing hinge (2) which enables the handle (8) to be positioned in different points relative to the body and which fixes it to the body (1) in a desired position.

4. A reamer handle according to claim 1, **characterized in that** said adjustment lever lock with radius (12) is positioned on said handle (8).

5. A reamer handle according to claims 1, **characterized in that** the angle adjustment capability of the motor connection shaft (3) is between 29° and 67° on the horizontal plane and between 29° and 81° on the vertical plane relative to the body axis of the apparatus and **in that** the adjustment capability of the motor connection shaft via the adjustment lever with radius is between 43° and 53,5° in the horizontal plane and between 43° and 63,5° in the vertical plane.

6. A reamer handle according to claim 1, **characterized in that** it comprises motor adaptors (6) to be mounted to the motor connection shaft (3) in order to provide connections with different motors.

7. A reamer handle according to claim 1, **characterized in that** it comprises a channel (10) formed on said handle (8) and/or on said stable transfer member (4).

8. A reamer handle according to claim 3, **characterized in that** said handle fixing hinge (2) is located on said holder (9).

## Patentansprüche

1. Ein Reibahle Griff für die Übertragung des Drehmomentes vom chirurgischen Motor auf eine Reibahle, die für das Aufbohren des Beckens (acetabulum) benutzt wird, durch einen Verbindungskopf der Reibahle (11), der Griff kann für das Behandeln von den Reibahlen von verschiedenem Durchmesser, die in Abhängigkeit von der Knochengröße gewechselt werden können, für das Bohren des Beckens (acetabulum) in der minimalen invasiven orthopädischen Chirurgie; besteht aus einem Körper (1); aus einem Verbindungskopf der Reibahle (11), mit dem die Reibahle für das Benutzen innerhalb des menschlichen Körpers verbunden werden kann; aus einem Übertragungsmitglied der stabilen Bewegung (4), das den Körper (1) mit dem Verbindungskopf der Reibahle (11) verknüpft, und das für die Übertragung des Drehmomentes vom Motor zum oben genannten Verbindungskopf der Reibahle (11) konfiguriert ist; aus einer Verbindungswelle des Motors, die die Übertragung des Drehmomentes vom Motor auf das oben genanntes Übertragungsmitglied der stabilen Bewegung (4) durchführt; aus einem beweglichen Scharnier (5), das die Verwendung von der Verbindungswelle des Motors (3) bei dem normalen Freiwinkel in der vertikalen und der horizontalen Ebenen relativ zur Körperachse des Operierten für die Übertragung des Drehmomentes von der oben genannten Verbindungswelle des Motors (3) unter verschiedenen Winkeln auf das Übertragungsmitglied der stabilen Bewegung (4) ermöglicht; aus einem Griff (8), der mit dem genannten Körper (1) verbunden ist, und der das Halten des Reibahlensgriffs die Achsen entlang, die sich von der Körperachse unterscheiden, ermöglichtet, und das mit der genannten Verbindungswelle des Motors verbunden werden kann (3); aus einem Justierungshebel mit dem Radius (7), der auf dem genannten Griff (8) platziert ist und für die Vergrößerung der Bewegungsleichtigkeit der genannten Verbindungswelle des Motors (3) dient, der Justierungshebel (7) ist abnehmbar und sein Ende außerhalb des Griffs (8) kann mit der Verbindungswelle des Motors (3) verknüpft werden; **dadurch gekennzeichnet, dass** der Justierungshebel (7) den Schloss für Justierungshebel mit dem Radius (12) beinhaltet, der die Fixierung des Justierungshebels (7) in der gewünschten Position ermöglicht.

2. Ein Reibahle Griff nach Anspruch 1 gekennzeichnet, dass ein Reibahle Griff mit dem genannten Körper (1) verbundenen Halter (9) beinhaltet.

3. Ein Reibahle Griff nach Anspruch 1 gekennzeichnet, dass ein Reibahle Griff für die das Befestigungsscharnier (2) beinhaltet, das die Positionierung des Griffs (8) in verschiedenen Stellen relativ zu menschlichem Körper ermöglicht und diesen Griff in der gewünschten Position auf dem Körper (1) fixiert.

4. Ein Reibahle Griff nach Anspruch 1 gekennzeichnet, dass das dort genannte Schloss des Justierungshebels mit dem Radius (12) auf dem genannten Griff (8) platziert ist.

5. Ein Reibahle Griff nach Anspruch 1 gekennzeichnet, dass die Winkeljustierungsmöglichkeit der Verbindungswelle des Motors (3) zwischen Winkeln 29 und 67 in der horizontalen Ebene und zwischen Winkeln 29 und 81 in der vertikalen Ebene relativ zur Körperachse des Operierten eingeschlossen ist, und die Winkeljustierungsmöglichkeit der Verbindungswelle des Motors mittels Justierungshebel mit dem Radius ist zwischen den Winkeln 43 und 53,5 in der horizontalen Ebene und zwischen Winkeln 43 und 63,5 in der vertikalen Ebene eingeschlossen.

6. Ein Reibahle Griff nach Anspruch 1 gekennzeichnet, dass ein Reibahle Griff die Motorenadapter (6) beinhaltet, die auf die Verbindungswelle des Motors (3) montiert werden können, um die Verwendung von verschiedenen Motoren zu ermöglichen.

7. Ein Reibahle Griff nach Anspruch 1 gekennzeichnet, dass ein Reibahle Griff den Kanal (10) beinhaltet, der in dem genannten Griff (8) und/oder im Übertragungsmitglied der stabilen Bewegung (4) geformt ist.

8. Ein Reibahle Griff nach Anspruch 3 gekennzeichnet, dass sich das Befestigungsscharnier (2) auf dem Halter (9) befindet.

## Revendications

1. Une poignée de fraise pour transmettre le mouvement d'un moteur chirurgical vers une fraise utilisée pour fraiser le pelvis (acétabulum) par une tête de raccordement de fraise (11), afin d'être utilisée pour actionner les fraises de différents diamètres, variant selon la taille de l'os, pour fraiser le pelvis (acétabulum) en chirurgie orthopédique invasive limitée; qui comprend un corps (1); une tête de raccordement de fraise (11) à laquelle une fraise destinée à être utilisée à l'intérieur du corps humain peut être connectée; un élément stable de transfert de mouvement qui raccorde le corps (1) et la tête de raccordement de fraise (11) et qui est configuré pour transférer le mouvement du moteur vers ladite tête de raccordement de fraise (11); une barre de liaison du moteur (3) pour transmettre le mouvement du moteur vers ledit élément stable de transfert de mouvement (4); une charnière mobile permettant à la barre de liaison du moteur (3) d'être utilisé à un angle de dépouille normal dans le plan vertical et le plan horizontal par rapport à l'axe du corps de l'appareil pour transmettre le mouvement de ladite barre de liaison du moteur (3) à des valeurs angulaires différentes vers l'élément stable de transfert de mouvement (4); une poignée (8) qui est raccordée audit corps (1) et qui permet à la poignée de fraise de se tiendra le long d'un axe différent de l'axe du corps et qui peut être raccordée sur ladite barre de liaison du moteur (3); un levier de réglage ayant un rayon (7) qui est placé dans ladite poignée (8) afin d'augmenter la facilité de mouvement de ladite barre de liaison du moteur (3) et qui est déplaçable et dont l'extrémité à extérieure de la poignée (8) peut être raccordée à ladite barre de liaison du moteur (3); **caractérisée en ce qu'**il comprend un verrou de levier de réglage ayant un rayon (12) permettant audit levier de réglage ayant un rayon (7) d'être fixé dans une position désirée.

2. Une poignée de fraise selon la revendication 1, **caractérisée en ce qu'**il comprend un porteur (9) raccordé audit corps (1).

3. Une poignée de fraise selon la revendication 1, **caractérisée en ce qu'**il comprend une charnière fixant en place la poignée (2) ce qui permet à la poignée (8) d'être positionné dans des points différents par rapport au corps et qui le fixe sur le corps (1) dans une position désirée.

4. Une poignée de fraise selon la revendication 1, **caractérisée en ce qu'**il comprend ledit verrou de levier de réglage ayant un rayon (12) est positionné sur ladite poignée (8).

5. Une poignée de fraise selon la revendication 1, **caractérisée en ce qu'**il comprend la capacité d'ajustement angulaire de ladite barre de liaison du moteur (3) se situe entre 29° et 67° sur le plan horizontal et entre 29° et 81° sur le plan vertical par rapport à l'axe du corps de l'appareil et **en ce que** la capacité d'ajustement de la barre de liaison du moteur au moyen du levier de réglage ayant un rayon se situe entre 43° et 53,5° dans le plan horizontal et entre 43° et 63,5° dans le plan vertical.

6. Une poignée de fraise selon la revendication 1, **caractérisée en ce qu'**il comprend des adaptateurs de moteur (6) destinés à être montés sur la barre de liaison du moteur (3) afin de fournir des interconnexions avec des moteurs différents.

7. Une poignée de fraise selon la revendication 1, **caractérisée en ce qu'**il comprend un canal (10) formé sur ladite poignée (8) et/ou sur ledit élément stable de transfert (4).

8. Une poignée de fraise selon la revendication 3, **caractérisée en ce que** ladite charnière fixant en place la poignée (2) est placée sur ledit porteur (9).
